# EUROPÄISCHE PATENTSCHRIFT

(11) **EP 4 279 016 B1**
(45) Veröffentlichungstag und Bekanntmachung des Hinweises auf die Patenterteilung: **29.01.2025**
(21) Anmeldenummer: 23165756.0
(22) Anmeldetag: 31.03.2023
(51) Int. Cl.: A61B 90/13, A61B 34/30, A61B 5/055, A61B 6/03, A61B 90/00, G09B 23/28, A61B 17/34, A61B 34/20, A61B 6/00

(54) **VORRICHTUNG UND VERFAHREN ZUM AUSRICHTEN EINES MEDIZINISCHEN OBJEKTS**
DEVICE AND METHOD FOR ALIGNING A MEDICAL OBJECT
DISPOSITIF ET PROCÉDÉ D'ALIGNEMENT D'UN OBJET MÉDICAL

(30) Priorität: 17.05.2022 DE 102022204859
(43) Veröffentlichungstag der Anmeldung: 22.11.2023
(73) Patentinhaber: Siemens Healthineers AG, 91301 Forchheim (DE)
(72) Erfinder: Regensburger, Alois, 91099 Poxdorf (DE); Hornung, Oliver, 91364 Unterleinleiter (DE)
(74) Vertreter: Siemens Healthineers Patent Attorneys

(56) Entgegenhaltungen:
- WO-A1-2018/200799
- DE-A1- 102018 215 599
- DE-T2- 69 620 521
- DE-T2- 69 728 908
- DE-T2- 69 829 161
- US-A1- 2016 206 383
- US-A1- 2019 269 470

## Beschreibung

Die Erfindung betrifft eine Vorrichtung zum Ausrichten eines medizinischen Objekts bezüglich eines Untersuchungsobjekts, ein System und ein Verfahren zum Ausrichten eines medizinischen Objekts.

Zur Ausrichtung medizinischer Objekte, beispielsweise einer Nadel, werden häufig sogenannte Nadel-Hülsenhalter verwendet, welche einen Hohlzylinder mechanisch stabil ausrichten, durch welchen das medizinische Objekt, insbesondere die Nadel, vorgeschoben werden kann. Hierdurch kann ein geplanter Pfad für das medizinische Objekt, insbesondere die Nadel, präzise vorgegeben werden, insbesondere mit höherer Genauigkeit als bei einer freihändigen Ausrichtung des medizinischen Objekts. Zur Ausrichtung der Hülse kann beispielsweise eine optische Navigation oder ein Roboter verwendet werden. Beispielsweise können mittels eines Lasers eine Einstichstelle für das medizinische Objekt, insbesondere die Nadel, auf dem Untersuchungsobjekt vorgegeben werden. Nach einer, insbesondere manuellen, Positionierung einer Spitze des medizinischen Objekts an der durch den Laser vorgegebenen Einstichstelle kann ein proximaler Abschnitt des medizinischen Objekts, insbesondere manuell, derart repositioniert werden, dass auch der proximale Abschnitt auf einem durch den Laser vorgegebenen Pfad angeordnet ist. Nachteilig ist dieses Verfahren zur Ausrichtung des medizinischen Objekts häufig unpräzise, schmerzhaft und kann zu einem erhöhten Blutverlust des Untersuchungsobjekts führen.

Zudem existieren verschiedene Ausrichthilfen, welche eine intuitivere Ausrichtung der Hülse bezüglich des Lesers ermöglichen können, indem die Ausrichthilfe an der Hülse befestigt und/oder anstelle des medizinischen Objekts in die Hülse eingeführt wird. DE102018215599A1 offenbart ein Ausrichtelement zum Ausrichten einer Nadelführung, welche zur Längsführung einer medizinischen Nadel eingerichtet ist. Es umfasst ein Verbindungselement zum Anordnen des Ausrichtelements an einer solchen Nadelführung, und eine Lichtführungseinrichtung für eine vorbestimmte Lichtverteilung, wobei die Lichtführungseinrichtung nur bei einer durch deren Geometrie vorgegebenen Pose relativ zu der Lichtverteilung ein vorbestimmtes Lichtmuster erzeugt.

Trotz der Verwendung einer Ausrichthilfe kann das Ausrichten der Hülse aufgrund der vielen räumlichen Freiheitsgrade zeitintensiv und aufwendig sein.

Es ist daher die Aufgabe der vorliegenden Erfindung, eine zeiteffiziente, präzise und intuitive Ausrichtung eines medizinischen Objekts in einer definierten Lagebeziehung bezüglich eines vorgegebenen Pfads zu ermöglichen.

Die Aufgabe wird erfindungsgemäß gelöst durch den jeweiligen Gegenstand der unabhängigen Ansprüche. Vorteilhafte Ausführungsformen mit zweckmäßigen Weiterbildungen sind Gegenstand der Unteransprüche.

Die Erfindung betrifft in einem ersten Aspekt eine Vorrichtung zum Ausrichten eines medizinischen Objekts bezüglich eines Untersuchungsobjekts, umfassend ein Ausrichtelement, ein Führungselement, ein Hilfselement und eine Lichtführungseinrichtung. Das Ausrichtelement ist zwangsgeführt durch das Führungselement um ein Rotationszentrum herum beweglich gelagert, welches Rotationszentrum durch Repositionieren des Führungselements repositionierbar ist. Das Ausrichtelement ist zur Längsführung des medizinischen Objekts entlang einer durch das Ausrichtelement vorgegebenen Führungsachse ausgebildet. Ferner weist das Hilfselement eine Erkennungsmarke auf. Das Hilfselement ist in einer definierten Anordnung bezüglich des Ausrichtelements derart anordbar, dass die Erkennungsmarke in dem Rotationszentrum angeordnet ist. Die Lichtführungseinrichtung ist dazu ausgebildet, eine vordefinierte Lichtverteilung auszusenden, welche einen Pfad vorgibt. Die vordefinierte Lichtverteilung beleuchtet die Erkennungsmarke bei einer von dem Untersuchungsobjekt beabstandeten Anordnung des Rotationszentrums auf dem Pfad. Dabei ist die Anordnung von Ausrichtelement und Hilfselement um das in einem Betriebszustand der Vorrichtung auf dem Pfad angeordnete Rotationszentrum derart bewegbar sein, dass die vordefinierte Lichtverteilung die Erkennungsmarke mit einem vordefinierten Lichtmuster beleuchtet, wenn die Führungsachse des Ausrichtelements in einer definierten Lagebeziehung bezüglich des Pfads ausgerichtet ist.

Das medizinische Objekt kann ein chirurgisches Instrument, beispielsweise eine Nadel, insbesondere eine Punktionsnadel, und/oder ein Bohrer, und/oder ein diagnostisches Instrument, beispielsweise ein Endoskop, insbesondere ein Laparoskop, und/oder ein Katheter, sein. Vorteilhafterweise kann das medizinische Objekt zumindest teilweise, insbesondere vollständig, starr und langgestreckt, insbesondere stab- und/oder nadelförmig, ausgebildet sein.

Das Untersuchungsobjekt kann beispielsweise eine menschliche und/oder tierische Patientin und/oder ein menschlicher und/oder tierischer Patient und/oder ein Untersuchungsphantom sein.

Das Ausrichtelement kann dazu ausgebildet sein, das medizinische Objekt entlang der Führungsachse, insbesondere auf der Führungsachse, zu führen. Insbesondere kann das Ausrichtelement dazu ausgebildet sein, Bewegungsfreiheitsgrade des medizinischen Objekts auf eine Translation entlang der Führungsachse und eine Rotation um die Führungsachse herum zu beschränken. Hierfür kann das Ausrichtelement dazu ausgebildet sein, das medizinische Objekt zumindest teilweise aufzunehmen und das medizinische Objekt durch eine, insbesondere mechanische und/oder elektromagnetische, Wechselwirkung des Ausrichtelements mit dem medizinischen Objekt entlang der Führungsachse zu führen. Die Führungsachse kann dabei ortsfest bezüglich des Ausrichtelements, beispielsweise durch eine Geometrie des Ausrichtelements, vorgegeben sein.

Das Führungselement kann vorteilhafterweise dazu ausgebildet sein, Bewegungsfreiheitsgrade des Ausrichtelements auf eine Rotation um das Rotationszentrum herum zu beschränken. Das Führungselement kann beispielsweise eine Haltevorrichtung, insbesondere ein Stativ und/oder einen Roboterarm, umfassen, an welchem das Ausrichtelement um das Rotationszentrum herum beweglich gelagert ist. Dabei kann das Rotationszentrum durch das Führungselement, insbesondere eine Geometrie des Führungselements, vorgegeben sein. Das Rotationszentrum kann durch eine Repositionierung des Führungselements, insbesondere eine Translation zumindest eines Teils des Führungselements, repositionierbar, insbesondere translatierbar, sein. Dabei kann das Führungselement dazu ausgebildet sein, manuell, beispielsweise durch eine Krafteinwirkung eines medizinischen Bedienpersonals, und/oder motorisch, beispielsweise mittels eines Motorantriebs, repositioniert zu werden. Das Ausrichtelement kann an dem Führungselement befestigt sein, wobei das Ausrichtelement durch eine Bewegung zumindest eines Teils des Führungselements bewegbar, insbesondere rotierbar, um das Rotationszentrum gelagert sein kann. Das Führungselement kann ferner dazu ausgebildet sein, einen Radius der Bewegung, insbesondere der Rotation, des Ausrichtelements um das Rotationszentrum vorzugeben.

Das Rotationszentrum kann eine räumliche Position beschreiben, um welche das Ausrichtelement zwangsgeführt durch das Führungselement bewegbar, insbesondere rotierbar, gelagert ist. Dabei kann das Rotationszentrum an dem Ausrichtelement oder von dem Ausrichtelement beabstandet angeordnet sein.

Das Hilfselement weist die Erkennungsmarke auf. Dabei kann die Erkennungsmarke als graphische Markierung, beispielsweise ein graphisches Objekt und/oder ein Muster, auf einer Oberfläche des Hilfsobjekts ausgebildet sein. Alternativ oder zusätzlich kann die Erkennungsmarke als strukturelles Element, beispielsweise eine Aussparung, insbesondere ein Schlitz, und/oder eine Erhebung, des Hilfselements ausgebildet sein. Die Erkennungsmarke kann insbesondere eine erste und eine zweite Teilerkennungsmarke umfassen. Dabei kann die erste Teilerkennungsmarke einen Punkt des Hilfselements markieren, welcher in dem Rotationszentrum anzuordnen ist. Die erste und die zweite Teilerkennungsmarke können eine definierte, insbesondere unveränderliche, Anordnung zueinander aufweisen, insbesondere eine Relativpositionierung. Die Anordnung der ersten und zweiten Teilerkennungsmarke kann eine Längsachse des Hilfselements definieren, welche auf dem vorgegebenen Pfad anzuordnen ist. Dabei kann die Erkennungsmarke, insbesondere die erste und die zweite Teilerkennungsmarke, dazu ausgebildet sein, bei der Anordnung der Längsachse des Hilfselements auf dem Pfad, mit dem vordefinierten Lichtmuster beleuchtet zu werden. Die erste und die zweite Teilerkennungsmarke können voneinander beabstandet oder zumindest teilweise zusammenhängend ausgebildet sein. Ferner können die erste und die zweite Teilerkennungsmarke jeweils als graphische Markierung auf einer Oberfläche des Hilfselements, insbesondere auf verschiedenen Oberflächen oder derselben Oberfläche des Hilfselements, und/oder als strukturelles Element des Hilfselements ausgebildet sein.

Vorteilhafterweise kann das Hilfselement dazu ausgebildet sein, in der definierten Anordnung bezüglich des Ausrichtelements angeordnet zu werden, insbesondere in einer Relativpositionierung bezüglich des Ausrichtelements. Insbesondere kann das Hilfselement an dem Ausrichtelement, insbesondere ortsfest, in der definierten Anordnung angeordnet sein und/oder in der definierten Anordnung in das Ausrichtelement integriert sein. Alternativ kann das Hilfselement bezüglich des Ausrichtelements repositionierbar sein, insbesondere translatierbar und/oder rotierbar. Beispielsweise kann das Hilfselement dazu ausgebildet sein, in die definierte Anordnung bezüglich des Ausrichtelements repositioniert zu werden. Ferner kann das Hilfselement dazu ausgebildet sein, in der definierten Anordnung an dem Ausrichtelement befestigt zu werden und/oder zur definierten Anordnung zumindest teilweise in das Ausrichtelement eingeführt zu werden.

Vorteilhafterweise kann die Erkennungsmarke, insbesondere die erste Teilerkennungsmarke, derart an dem Hilfselement angeordnet sein, dass die Erkennungsmarke, insbesondere die erste Teilerkennungsmarke, in dem Rotationszentrum angeordnet ist, wenn das Hilfselement bezüglich des Ausrichtelements in der definierten Anordnung angeordnet ist.

Die Lichtführungseinrichtung kann vorteilhafterweise eine Lichtquelle, beispielsweise eine Laserlichtquelle, umfassen, welche dazu ausgebildet ist, die vordefinierte Lichtverteilung auszusenden. Hierfür kann die Lichtführungseinrichtung beispielsweise eine optische Blende umfassen. Die vordefinierte Lichtverteilung kann vorteilhafterweise eine übliche Lichtverteilung einer üblichen Lichtquelle zum Darstellen eines Pfads, insbesondere eines Nadelpfads, sein. Insbesondere kann die vordefinierte Lichtverteilung eine vordefinierte Verteilung von Laserlicht umfassen. Dabei kann die vordefinierte Lichtverteilung ein vordefiniertes Lichtmuster projizieren, beispielsweise überkreuzende Linien und/oder einen Punkt. Durch die vordefinierte Lichtverteilung kann der vorgegebene Pfad, bezüglich welchem das medizinische Objekt in der definierten Lagebeziehung auszurichten ist, projiziert werden. Das vordefinierte Lichtmuster kann durch Reflektieren und/oder Absorbieren zumindest eines Teils der vordefinierten Lichtverteilung durch die Erkennungsmarke erzeugt, insbesondere bereitgestellt, werden.

Vorteilhafterweise kann die vordefinierte Lichtverteilung, insbesondere ein Schnittpunkt der projizierten überkreuzenden Linien und/oder der projizierte Punkt, bei Anordnung des Rotationszentrums auf dem Pfad die Erkennungsmarke, insbesondere die erste Teilerkennungsmarke, beleuchten. Insbesondere kann die vordefinierte Lichtverteilung, insbesondere ein Schnittpunkt der projizierten überkreuzenden Linien und/oder der projizierte Punkt, genau dann die Erkennungsmarke, insbesondere die erste Teilerkennungsmarke, beleuchten, wenn das Rotationszentrum auf dem Pfad angeordnet ist. Zur Anordnung des Rotationszentrums auf dem Pfad kann das Führungselement repositionierbar ausgebildet sein, beispielsweise translatierbar und/oder rotierbar. Vorteilhafterweise kann die definierte Anordnung von Ausrichtelement und Hilfselement bei dem Repositionieren des Führungselements unbeeinflusst bleiben.

Die Anordnung, insbesondere die definierte Anordnung oder eine weitere Anordnung, von Ausrichtelement und Hilfselement können um das in einem Betriebszustand der Vorrichtung auf dem Pfad angeordnete Rotationszentrum derart bewegbar, insbesondere rotierbar, sein, dass die vordefinierte Lichtverteilung, insbesondere genau dann, die Erkennungsmarke mit dem vordefinierten Lichtmuster beleuchtet, wenn die Führungsachse des Ausrichtelements in der definierten Lagebeziehung bezüglich des Pfads ausgerichtet ist. Die definierte Lagebeziehung kann dabei eine Relativpositionierung zwischen der Führungsachse des Ausrichtelements und dem vorgegebenen Pfad beschreiben. Insbesondere kann die definierte Lagebeziehung eine parallele Ausrichtung der Führungsachse des Ausrichtelements bezüglich des vorgegebenen Pfads und/oder eine Ausrichtung der Führungsachse des Ausrichtelements entlang des vorgegebenen Pfads vorgeben. Alternativ kann die definierte Lagebeziehung eine Ausrichtung der Führungsachse des Ausrichtelements in einem vorgegebenen Winkel, insbesondere einem Schnittwinkel zwischen Führungsachse und vorgegebenem Pfad oder einem Winkel in einer vorgegebenen räumlichen Distanz zwischen Führungsachse und vorgegebenem Pfad, bezüglich des vorgegebenen Pfads vorgeben.

Die vorgeschlagene Ausführungsform kann vorteilhaft eine zeiteffiziente, präzise und intuitive Ausrichtung des medizinischen Objekts in der definierten Lagebeziehung bezüglich des vorgegebenen Pfads ermöglichen.

In einer weiteren vorteilhaften Ausführungsform der vorgeschlagenen Vorrichtung kann das Ausrichtelement eine röhrenförmige Hülse aufweisen, welche dazu ausgebildet ist, das medizinische Objekt aufzunehmen und entlang der Führungsachse zu führen.

Die röhrenförmige Hülse kann im Wesentlichen zylindrisch ausgebildet sein. Insbesondere kann die röhrenförmige Hülse einen Mantel aus einem Substrat aufweisen, insbesondere mit einem runden oder kantigen Querschnitt, welcher einen Hohlraum umgibt. Die röhrenförmige Hülse kann ferner zwei Öffnungen entlang ihrer Längserstreckungsrichtung aufweisen, insbesondere an jeweils einer von zwei Stirnflächen. Dabei kann das medizinische Objekt durch die erste Öffnung der Hülse in den Hohlraum der Hülse, insbesondere des Mantels, einführbar und durch die zweite Öffnung der Hülse ausbringbar sein. Ferner kann die Hülse dazu ausgebildet sein, das medizinische Objekt, insbesondere durch mechanische Wechselwirkung mit dem Mantel der Hülse, entlang der Führungsachse zu führen. Bei einer im Wesentlichen zylindrischen Ausbildung der Hülse kann die Führungsachse parallel zu einer Längsachse, insbesondere einer Symmetrieachse, des Zylinders verlaufen.

Die vorgeschlagene Ausführungsform kann vorteilhaft ein sicheres und präzises Führen des medizinischen Objekts entlang der Führungsachse ermöglichen.

In einer weiteren vorteilhaften Ausführungsform der vorgeschlagenen Vorrichtung kann das Hilfselement zumindest einen scheibenförmigen Abschnitt aufweisen. Dabei kann die Erkennungsmarke auf einer flachen Seite des zumindest einen scheibenförmigen Abschnitts angeordnet sein und/oder in den zumindest einen scheibenförmigen Abschnitt integriert sein.

Der scheibenförmige Abschnitt kann aus einem Substrat und/oder im Wesentlichen flach ausgebildet sein. Dabei kann der scheibenförmigen Abschnitt zumindest eine flache Seite aufweisen. Vorteilhafterweise kann die zumindest eine flache Seite in der definierten Anordnung des Hilfselements der Lichtführungseinrichtung, insbesondere der Lichtquelle, zugewandt sein. Ferner kann der scheibenförmige Abschnitt, insbesondere die flache Seite des scheibenförmigen Abschnitts, in der definierten Anordnung abgewinkelt, insbesondere rechtwinklig, bezüglich einer Lichteinfallsrichtung der von der Lichtführungseinrichtung ausgesandten Lichtverteilung angeordnet sein, wenn die Führungsachse des Ausrichtelements in der definierten Lagebeziehung bezüglich des Pfads ausgerichtet ist. Vorteilhafterweise kann die Erkennungsmarke, insbesondere die erste und/oder die zweite Teilerkennungsmarke, auf der zumindest einen flachen Seite des zumindest einen scheibenförmigen Abschnitts angeordnet sein, beispielsweise als graphische Markierung, und/oder in den zumindest einen scheibenförmigen Abschnitt integriert sein, beispielsweise als strukturelles Element. Hierdurch kann die vordefinierte Lichtverteilung die zumindest eine flache Seite des scheibenförmigen Abschnitts bei einer Anordnung des Rotationszentrums auf dem Pfad beleuchten.

In einer weiteren vorteilhaften Ausführungsform der vorgeschlagenen Vorrichtung kann das Ausrichtelement zwangsgeführt durch das Führungselement um zumindest eine Rotationsachse, welche durch das Rotationszentrum verläuft, beweglich gelagert sein.

Das Führungselement kann vorteilhafterweise dazu ausgebildet sein, die Bewegungsfreiheitsgrade des Ausrichtelements auf eine Rotation um die zumindest eine Rotationsachse, insbesondere mehrere Rotationsachsen, zu beschränken, welche zumindest eine Rotationsachse durch das Rotationszentrum verläuft. Insbesondere kann das Führungselement dazu ausgebildet sein, eine Translation des Ausrichtelements bezüglich des Rotationszentrums durch die Zwangsführung zu sperren. Die zumindest eine Rotationsachse kann eine Raumachse beschreiben, welche durch das Rotationszentrum verläuft und um welche das Ausrichtelement beweglich, insbesondere rotierbar, gelagert ist.

Ist das Führungselement dazu ausgebildet, die Bewegungsfreiheitsgrade des Ausrichtelements auf eine Rotation um genau eine Rotationsachse zu beschränken, welche Rotationsachse durch das Rotationszentrum verläuft, kann das Ausrichtelement innerhalb einer Ebene, welche senkrecht zu der Rotationsachse und durch das Rotationszentrum verläuft, beweglich, insbesondere rotierbar, gelagert sein.

In einer weiteren vorteilhaften Ausführungsform der vorgeschlagenen Vorrichtung kann das Ausrichtelement zwangsgeführt durch das Führungselement, um zwei verschiedene Rotationsachsen, welche durch das Rotationszentrum verlaufen, beweglich gelagert sein.

Vorteilhafterweise können sich die zwei verschiedenen Rotationsachsen in dem Rotationszentrum schneiden. Dabei können die zwei verschiedenen Rotationsachsen einen vorgegebenen Winkel, beispielsweise einen rechten Winkel, zueinander in dem Rotationszentrum aufweisen. Das Führungselement kann dazu ausgebildet sein, die Bewegungsfreiheitsgrade des Ausrichtelements auf eine Rotation um die zwei verschiedenen Rotationsachsen zu beschränken. Das Ausrichtelement kann zwangsgeführt durch das Führungselement auf einer Kugelschale um das Rotationszentrum herum, insbesondere um die zwei verschiedenen Rotationsachsen herum, beweglich, insbesondere rotierbar, gelagert sein.

Das Führungselement kann beispielsweise als, insbesondere motorisierte, kardanische Aufhängung, beispielsweise ein Gimbal, ausgebildet sein. Die vorgeschlagene Ausführungsform kann ein besonders flexibles, insbesondere dreidimensionales, Repositionieren der definierten Anordnung von Ausrichtelement und Hilfselement um das Rotationszentrum herum ermöglichen.

In einer weiteren vorteilhaften Ausführungsform der vorgeschlagenen Vorrichtung kann die Lichtführungseinrichtung dazu ausgebildet sein, die vordefinierte Lichtverteilung aufweisend mehrere Lichtfächer auszusenden, welche nicht parallel zueinander verlaufen und sich entlang des Pfads schneiden.

Die Lichtführungseinrichtung kann dazu ausgebildet sein, die mehreren Lichtfächer in mehreren Ebenen durch Aussenden der vordefinierten Lichtverteilung zu erzeugen, wobei sich die mehreren Ebenen entlang des Pfads schneiden und nicht parallel zueinander verlaufen. Eine Schnittgerade der mehreren Ebenen, insbesondere der mehreren Lichtfächer, kann den vorgegebenen Pfad bilden. Dabei kann jeweils eine der mehreren Lichtfächer durch mehrere Lichtstrahlen gebildet werden, welche jeweils innerhalb einer der mehreren Ebenen verlaufen, beispielsweise fächerförmig und/oder parallel zueinander. Durch das Aussenden der vordefinierten Lichtverteilung, aufweisend die mehreren Lichtfächer, können überkreuzende Linien von der Lichtführungseinrichtung projiziert werden. Dabei kann ein Schnittpunkt der überkreuzenden Linien auf dem Pfad liegen, insbesondere den Pfad markieren. Hierfür kann die Lichtführungseinrichtung beispielsweise zwei gekreuzte Linienlaser umfassen.

Insbesondere kann die Lichtführungseinrichtung dazu ausgebildet sein, die vordefinierte Lichtverteilung, aufweisend zwei Lichtfächer, auszusenden, welche Lichtfächer rechtwinklig zueinander verlaufen und sich entlang des Pfads schneiden. Hierdurch kann ein präzises und gut erfassbares Vorgeben des Pfads durch die vordefinierte Lichtverteilung ermöglicht werden. Insbesondere kann, bei der Anordnung der Führungsachse des Ausrichtelements in der definierten Lagebeziehung bezüglich des Pfads, die vordefinierte Lichtverteilung das vordefinierte Lichtmuster durch die mehreren Lichtfächer auf die Erkennungsmarke projizieren.

In einer weiteren vorteilhaften Ausführungsform der vorgeschlagenen Vorrichtung kann die Vorrichtung weiterhin zumindest ein selektiv aktivierbares Feststellelement umfassen. Das Feststellelement kann dazu ausgebildet sein, bei Aktivierung die Repositionierung des Rotationszentrums zu sperren. Alternativ oder zusätzlich kann das zumindest eine Feststellelement dazu ausgebildet sein, bei Aktivierung eine Beweglichkeit des Ausrichtelements auf eine Rotation um eine Rotationsachse, welche durch das Rotationszentrum verläuft, zu beschränken.

Das zumindest eine Feststellelement kann dazu ausgebildet sein, manuell, halbautomatisch und/oder vollautomatisch aktiviert zu werden, insbesondere wenn das Rotationszentrum auf dem vorgegebenen Pfad angeordnet ist. Für eine manuelle und/oder halbautomatische Aktivierung kann das zumindest eine Feststellelement ein Eingabemittel, beispielsweise eine Taste und/oder einen Knopf und/oder einen Schalter und/oder ein Pedal und/oder einen Hebel, und/oder ein, insbesondere mechanisches, Arretierungsmittel umfassen, beispielsweise eine Feststellschraube und/oder eine Schraubenmutter und/oder ein Klemmhebel, welches durch ein medizinisches Bedienpersonal aktivierbar, insbesondere manipulierbar, ist. Alternativ oder zusätzlich kann die Vorrichtung dazu ausgebildet sein, insbesondere automatisch, zu erfassen, ob das Rotationszentrum auf dem Pfad angeordnet ist, und bejahendenfalls das zumindest eine Feststellelement, beispielsweise elektromagnetisch und/oder motorisch und/oder robotisch, zu aktivieren. Hierfür kann die Vorrichtung, insbesondere das Hilfselement, einen Sensor, beispielsweise einen optischen und/oder elektromagnetischen Sensor, beispielsweise einen Photosensor, umfassen, welcher dazu ausgebildet ist, zu erfassen, ob die Erkennungsmarke mit der vordefinierten Lichtverteilung beleuchtet wird. Hierfür kann der Sensor beispielsweise in die Erkennungsmarke integriert und/oder an der Erkennungsmarke angeordnet sein.

Weist das Führungselement mehrere Bewegungsachsen, beispielsweise Gelenke und/oder Scharniere, auf, kann das zumindest eine Feststellelement vorteilhafterweise dazu ausgebildet sein, zumindest eine der Bewegungsachsen, insbesondere mehrere der Bewegungsachsen, bei Aktivierung zu sperren. Des Weiteren kann das zumindest eine Feststellelement dazu ausgebildet sein, manuell, halbautomatisch und/oder vollautomatisch deaktiviert zu werden, insbesondere wenn das Rotationszentrum nicht auf dem Pfad angeordnet ist. Hierbei kann das zumindest eine Feststellelement dazu ausgebildet sein, bei Deaktivierung die Repositionierung des Rotationszentrum, insbesondere des Führungselements, freizugeben.

Vorteilhafterweise kann das zumindest eine Feststellelement dazu ausgebildet sein, bei Aktivierung nur das Repositionieren des Rotationszentrums, insbesondere des Führungselements, zu sperren, wobei weiterhin eine Rotation des Ausrichtelements um das Rotationszentrum herum, insbesondere uneingeschränkt, möglich ist.

Alternativ oder zusätzlich kann das zumindest eine Feststellelement dazu ausgebildet sein, bei Aktivierung die Beweglichkeit des Ausrichtelements auf eine Rotation um eine Rotationsachse, welche durch das Rotationszentrum verläuft, zu beschränken. Vorteilhafterweise kann die Vorrichtung dazu ausgebildet sein, zu erfassen, ob das Rotationszentrum auf dem Pfad angeordnet ist, beispielsweise mittels des Sensors, und bejahendenfalls die Beweglichkeit des Ausrichtelements auf eine Rotation um eine Rotationsachse, welche durch das Rotationszentrum, insbesondere senkrecht zu dem Pfad, verläuft, zu beschränken. Dabei kann das zumindest eine Feststellelement dazu ausgebildet sein, bei Deaktivierung die Beweglichkeit des Ausrichtelements freizugeben.

Die vorgeschlagene Ausführungsform kann vorteilhaft die Bewegbarkeit der Anordnung von Ausrichtelement und Hilfselement um Rotationszentrum ermöglichen, insbesondere sicherstellen.

In einer weiteren vorteilhaften Ausführungsform der vorgeschlagenen Vorrichtung kann das Hilfselement in der definierten Anordnung an dem Ausrichtelement befestigt und/oder in das Ausrichtelement integriert sein.

Vorteilhafterweise kann das Hilfselement in der definierten Anordnung an dem Ausrichtelement, insbesondere lösbar, befestigt sein. Beispielsweise kann das Hilfselement in der definierten Anordnung mechanisch und/oder elektromagnetisch an das Ausrichtelement gekoppelt sein.

Alternativ oder zusätzlich kann das Hilfselement in das Ausrichtelement integriert sein. Beispielsweise kann das Ausrichtelement die Erkennungsmarke aufweisen, welche durch die vordefinierte Lichtverteilung beleuchtbar ist, und in der definierten Anordnung bezüglich des Ausrichtelements angeordnet ist. Vorteilhafterweise kann die Erkennungsmarke, insbesondere in einer scheibenförmigen Ausbildung, abgewinkelt bezüglich der Führungsachse in der definierten Anordnung angeordnet sein.

Mittels der vorgeschlagenen Ausführungsform kann vorteilhafterweise sichergestellt werden, dass die definierte Anordnung auch bei einer Bewegung des Ausrichtelements um das Rotationszentrum herum erhalten bleibt.

In einer weiteren vorteilhaften Ausführungsform der vorgeschlagenen Vorrichtung kann das Hilfselement längsgeführt in der definierten Lagebeziehung bezüglich der Führungsachse von der definierten Anordnung in eine weitere Anordnung repositionierbar sein. Dabei kann die weitere Anordnung von Ausrichtelement und Hilfselement um das Rotationszentrum herum derart bewegbar sein, dass die vordefinierte Lichtverteilung, insbesondere genau dann, die Erkennungsmarke mit dem vordefinierten Lichtmuster beleuchtet, wenn die Führungsachse des Ausrichtelements in der definierten Lagebeziehung bezüglich des Pfads ausgerichtet ist.

Vorteilhafterweise kann das Hilfselement durch eine, insbesondere mechanische und/oder elektromagnetische, Kopplung, insbesondere mit dem Führungselement und/oder dem Ausrichtelement, in der definierten Lagebeziehung bezüglich der Führungsachse längsgeführt bewegbar, insbesondere translatierbar, gelagert sein. Hierfür kann das Hilfselement ein erstes Kopplungselement aufweisen, beispielsweise eine Aussparung, insbesondere eine Nut und/oder eine Hülse, und/oder eine Erhebung, beispielsweise eine Feder und/oder einen Stab und/oder Stift. Ferner können das Führungselement und/oder das Ausrichtelement ein zweites Kopplungselement aufweisen, beispielsweise eine Aussparung und/oder eine Erhebung. Das erste und das zweite Kopplungselement können dazu ausgebildet sein, zur mechanischen Kopplung des Hilfselements mit dem Führungselement und/oder dem Ausrichtelement in mechanischen Eingriff gebracht zu werden. Ferner können das erste und das zweite Kopplungselement dazu ausgebildet sein, eine Beweglichkeit, insbesondere Bewegungsfreiheitsgrade, des Hilfselements auf eine Bewegung, insbesondere Translation, des Hilfselements in der definierten Lagebeziehung bezüglich der Führungsachse von der definierten Anordnung in die weitere Anordnung zu beschränken.

Die weitere Anordnung des Hilfselements bezüglich des Ausrichtelements kann verschieden von der definierten Anordnung des Hilfselements bezüglich des Ausrichtelements sein. Insbesondere kann das Hilfselement manuell, halbautomatisch und/oder vollautomatisch von der definierten Anordnung in die weitere Anordnung repositionierbar sein. Beispielsweise kann das Hilfselement dazu ausgebildet sein, durch manuelles Angreifen durch ein medizinisches Bedienpersonal von der definierten Anordnung in die weitere Anordnung repositioniert zu werden. Alternativ oder zusätzlich kann das Hilfselement dazu ausgebildet sein, motorisch und/oder robotisch, insbesondere halbautomatisch und/oder vollautomatisch, von der definierten Anordnung in die weitere Anordnung repositioniert werden.

Durch das Repositionieren des Hilfselements von der definierten Anordnung, längsgeführt in der definierten Lagebeziehung bezüglich der Führungsachse, in die weitere Anordnung kann eine verbesserte, insbesondere intuitivere, Rotation der Anordnung von Ausrichtelement und Hilfselement um das Rotationszentrum herum ermöglicht werden.

In einer weiteren vorteilhaften Ausführungsform der vorgeschlagenen Vorrichtung kann das Hilfselement dazu ausgebildet sein, zumindest teilweise in die Hülse eingeführt zu werden. Dabei kann das Hilfselement in der definierten Anordnung zumindest teilweise in die Hülse eingeführt sein.

Das Hilfselement kann dazu ausgebildet sein, zumindest teilweise, insbesondere vollständig, in die Hülse, insbesondere einen Hohlraum innerhalb des Mantels der Hülse, eingeführt zu werden, insbesondere über die erste Öffnung der Hülse. Hierfür kann das Hilfselement einen ersten, insbesondere stabförmigen, Abschnitt aufweisen, wobei ein Außendurchmesser des ersten Abschnitts kleiner als ein Innendurchmesser des Mantels der Hülse ist. Der erste, insbesondere stabförmige, Abschnitt kann im Wesentlichen langgestreckt ausgebildet sein. Dabei kann eine Längsachse des ersten Abschnitts in der definierten Anordnung parallel zur Führungsachse des Ausrichtelements angeordnet sein. Die Erkennungsmarke kann eine Ebene definieren, auf welche die Lichtführungseinrichtung das definierte Lichtmuster in dem Betriebszustand der Vorrichtung, in welchem die Führungsachse des Ausrichtelements in der definierten Lagebeziehung bezüglich des Pfads angeordnet ist, projiziert. Dabei kann die Ebene in einem vorgegebenen Winkel, insbesondere einem rechten Winkel, bezüglich der Längsachse des ersten, insbesondere stabförmigen, Abschnitts des Hilfselements angeordnet sein. Weist das Hilfselement einen scheibenförmigen Abschnitt mit der Erkennungsmarke auf, so kann die Ebene parallel zu dem scheibenförmigen Abschnitt angeordnet sein. Hierdurch kann vorteilhaft sichergestellt werden, dass die Erkennungsmarke mit dem vordefinierten Lichtmuster genau dann beleuchtet wird, wenn die Führungsachse in der vordefinierten Lagebeziehung bezüglich des Pfads angeordnet ist.

In einer weiteren vorteilhaften Ausführungsform der vorgeschlagenen Vorrichtung können das Hilfselement und/oder das Ausrichtelement ein Positionierungsmittel aufweisen, welches ein über die definierte Anordnung weitergehendes Einführen des Hilfselements in die Hülse blockiert.

Das Hilfselement und das Ausrichtelement können jeweils ein Positionierungsmittel aufweisen, welches das über die definierte Anordnung weitergehende Einführen des Hilfselements in die Hülse, insbesondere mechanisch und/oder elektromagnetisch, blockiert. Alternativ kann das Hilfselement oder das Ausrichtelement ein Positionierungsmittel aufweisen, welches das über die definierte Anordnung weitergehende Einführen des Hilfselements in die Hülse, insbesondere mechanisch und/oder elektromagnetisch, blockiert. Das Positionierungsmittel kann als strukturelles Merkmal des Ausrichtelements und/oder des Hilfselements, beispielsweise als Aussparung und/oder Erhebung, ausgebildet sein. Insbesondere kann das Positionierungsmittel dazu ausgebildet sein, bei Erreichen der definierten Anordnung einen mechanischen Eingriff zwischen dem Hilfselement und dem Ausrichtelement herzustellen, welcher das weitergehende Einführen des Hilfselements in die Hülse mechanisch blockiert. Beispielsweise kann das Positionierungsmittel einen Anschlagpunkt und/oder Anstoßpunkt zwischen dem Hilfselement und dem Ausrichtelement bereitstellen, welcher das weitergehende Einführen des Hilfselements in die Hülse durch Anschlagen und/oder Anstoßen des Hilfselements mechanisch blockiert. Alternativ oder zusätzlich kann das Positionierungsmittel dazu ausgebildet sein, bei einem über die definierte Anordnung weitergehenden Einführen des Hilfselements in die Hülse eine elektromagnetische Kraft auf das Hilfselement auszuüben, welche einer Richtung zum Einführen des Hilfselements in die Hülse entgegengerichtet ist und das weitergehende Einführen des Hilfselements in die Hülse elektromagnetisch blockiert.

Hierdurch kann ein besonders intuitives Anordnen des Hilfselements in der definierten Anordnung bezüglich des Ausrichtelements ermöglicht werden.

In einer weiteren vorteilhaften Ausführungsform der vorgeschlagenen Vorrichtung kann das Hilfselement einen stabförmigen Abschnitt aufweisen, welcher dazu ausgebildet ist, zumindest teilweise in die Hülse eingeführt zu werden. Ferner kann das Hilfselement einen weiteren Abschnitt aufweisen, welcher das Positionierungsmittel des Hilfselements bildet und das über die definierte Anordnung weitergehende Einführen des Hilfselements in die Hülse mechanisch blockiert.

Der stabförmige Abschnitt kann im Wesentlichen zylindrisch und langgestreckt ausgebildet sein. Ferner kann der stabförmige Abschnitt einen Außendurchmesser aufweisen welcher kleiner als ein Innendurchmesser des Mantels der Hülse ist. Vorteilhafterweise kann der stabförmige Abschnitt dazu ausgebildet sein, zumindest teilweise, insbesondere vollständig, in die Hülse eingeführt werden, insbesondere entlang der Führungsachse und/oder parallel zur Führungsachse. Ferner kann das Hilfselement einen weiteren Abschnitt aufweisen, welcher das Positionierungsmittel des Hilfselements bildet. Der weitere Abschnitt kann beispielsweise eine Erhebung und/oder eine, insbesondere scheibenförmige, Form aufweisen, wobei ein Außendurchmesser des weiteren Abschnitts größer als der Innendurchmesser des Mantels der Hülse ist. Hierdurch kann der weitere Abschnitt das über die definierte Anordnung weitergehende Einführen des Hilfselements, insbesondere des stabförmigen Abschnitts, in die Hülse, insbesondere den Hohlraum der Hülse, mechanisch blockieren. Der weitere Abschnitt kann einen Anschlagpunkt und/oder eine Anschlagfläche aufweisen, welche in der definierten Anordnung des Hilfselements bezüglich des Ausrichtelements die Hülse, insbesondere den Mantel der Hülse, mechanisch kontaktiert, insbesondere berührt.

Hierdurch kann ein besonders intuitives Anordnen des Hilfselements in der definierten Anordnung bezüglich des Ausrichtelements ermöglicht werden.

In einer weiteren vorteilhaften Ausführungsform der vorgeschlagenen Vorrichtung können das Hilfselement und/oder das Ausrichtelement ein Anzeigemittel aufweisen, welches die definierte Anordnung des Hilfselements bezüglich des Ausrichtelements anzeigt.

Das Hilfselement und das Ausrichtelement können jeweils ein Anzeigemittel aufweisen. Alternativ kann das Hilfselement oder das Ausrichtelement ein Anzeigenmittel aufweisen. Das Anzeigemittel kann dazu ausgebildet sein, die definierte Anordnung des Hilfselements bezüglich des Ausrichtelements durch Ausgabe eines akustischen und/oder optischen und/oder haptischen Signals anzuzeigen. Das Anzeigemittel kann eine graphische Markierung, beispielsweise eine Linie und/oder einen Punkt und/oder ein Muster und/oder ein Kreuz, und/oder ein strukturelles Element, beispielsweise eine Aussparung und/oder eine Erhebung und/oder eine Kante, und/oder Leuchtmittel, beispielsweise eine LED, umfassen, welche dazu ausgebildet ist, die definierte Anordnung des Hilfselements bezüglich des Ausrichtelements anzuzeigen. Beispielsweise kann die graphische Markierung und/oder das strukturelle Element des Hilfselements bei der definierten Anordnung des Hilfselements bezüglich des Ausrichtelements in einer Flucht und/oder Überdeckung mit einer weiteren graphischen Markierung und/oder einem weiteren strukturellen Element des Ausrichtelements angeordnet sein. Alternativ oder zusätzlich kann das Leuchtmittel durch Aussenden eines Lichtsignals die definierte Anordnung anzeigen. Hierfür kann das Hilfselement und/oder das Ausrichtelement einen Positionierungssensor aufweisen, welcher dazu ausgebildet ist, zu erfassen, ob das Hilfselement in der definierten Anordnung bezüglich des Ausrichtelements angeordnet ist. Bejahendenfalls kann der Positionierungssensor ein Signal an das Anzeigeelement bereitstellen, woraufhin das Anzeigeelement die definierte Anordnung des Hilfselements bezüglich des Ausrichtelements anzeigt.

Ferner kann das Anzeigemittel als, insbesondere mechanische und/oder elektromagnetische, Bremse und/oder als, insbesondere mechanischer und/oder elektromagnetischer, Einrastpunkt zwischen dem Hilfselement und dem Ausrichtelement ausgebildet sein. Die Bremse kann beispielsweise bei Erreichen der definierten Anordnung eine Kraft auf das Hilfselement ausüben, welche einer Bewegungsrichtung des Hilfselements, insbesondere der Richtung zum Einführen des Hilfselements in die Hülse, entgegengerichtet ist. Ferner kann der Einrastpunkt dazu ausgebildet sein, bei einem Verlassen der definierten Anordnung durch das Hilfselement, insbesondere einem Wegbewegen des Hilfselements aus der definierten Anordnung heraus, eine Kraft auf das Hilfselement auszuüben, welche einer Bewegungsrichtung zum Verlassen der definierten Anordnung entgegengerichtet ist. Dabei kann das Anzeigemittel dazu ausgebildet sein, die definierte Anordnung des Hilfselements bezüglich des Ausrichtelements als haptisches Signal, insbesondere haptisches Feedback, anzuzeigen.

Hierdurch kann ein besonders intuitives Anordnen des Hilfselements in der definierten Anordnung bezüglich des Ausrichtelements ermöglicht werden.

Die Erfindung betrifft in einem zweiten Aspekt ein System, umfassend eine vorgeschlagene Vorrichtung zum Ausrichten eines medizinischen Objekts bezüglich eines Untersuchungsobjekts und eine Navigationseinheit. Dabei umfasst die Navigationseinheit ein medizinisches Bildgebungsgerät und/oder ein medizinisches Navigationssystem und/oder ein medizinisches Behandlungssystem. Ferner ist die Lichtführungseinrichtung an der Navigationseinheit angeordnet und/oder in die Navigationseinheit integriert.

Die Vorteile des vorgeschlagenen Systems entsprechen im Wesentlichen den Vorteilen der vorgeschlagenen Vorrichtung zum Ausrichten eines medizinischen Objekts bezüglich eines Untersuchungsobjekts. Hierbei erwähnte Merkmale, Vorteile oder alternative Ausführungsformen können ebenso auch auf die anderen beanspruchten Gegenstände übertragen werden und umgekehrt.

Das medizinische Bildgebungsgerät kann beispielsweise ein medizinisches Röntgengerät und/oder eine Magnetresonanztomographieanlage (MRT-Anlage) und/oder eine Computertomographieanlage (CT-Anlage) und/oder eine Positronenemissionstomographieanlage (PET-Anlage) und/oder ein Ultraschallgerät umfassen. Dabei kann das medizinische Bildgebungsgerät zur Aufnahme von medizinischen Bilddaten des Untersuchungsobjekts ausgebildet sein. Die Lichtführungseinrichtung kann an dem medizinischen Bildgebungsgerät angeordnet und/oder in das medizinische Bildgebungsgerät integriert sein.

Das medizinische Navigationssystem kann beispielsweise in das medizinische Bildgebungssystem und/oder das medizinische Behandlungssystem integriert sein. Alternativ kann das medizinische Navigationssystem separat, insbesondere eigenständig, realisiert sein. Das medizinische Navigationssystem kann einen, insbesondere optischen und/oder elektromagnetischen und/oder akustischen, insbesondere ultraschallbasierten, Sensor aufweisen, welcher dazu ausgebildet ist, das Untersuchungsobjekt zu erfassen, insbesondere zu orten und/oder zu verfolgen (engl. tracking). Insbesondere kann das medizinische Navigationssystem dazu ausgebildet sein, eine, insbesondere momentane, Positionierung, insbesondere eine Position und/oder Ausrichtung und/oder Pose, des Untersuchungsobjekts zu erfassen. Das medizinische Navigationssystem kann ferner dazu ausgebildet sein, die Navigationsdaten aufweisend eine Information zu der, insbesondere momentanen, Positionierung des Untersuchungsobjekts bereitzustellen. Die Lichtführungseinrichtung kann an dem medizinischen Navigationssystem angeordnet und/oder in das medizinische Navigationssystem integriert sein.

Das medizinische Behandlungssystem kann beispielsweise eine medizinische Bestrahlungsanlage und/oder ein therapeutisches Ultraschallgerät umfassen. Dabei kann die Lichtführungseinrichtung an dem medizinischen Behandlungssystem angeordnet und/oder in das medizinische Behandlungssystem integriert sein.

Das System, insbesondere die Navigationseinheit, kann dazu ausgebildet sein, das Aussenden der vorgegebenen Lichtverteilung durch die Lichtführungseinheit derart zu steuern, dass der vorgegebene Pfad anpassbar ist, beispielsweise in Abhängigkeit von mittels des medizinischen Bildgebungsgeräts aufgenommenen medizinischen Bilddaten und/oder von mittels des medizinischen Navigationssystems erfassten Navigationsdaten und/oder von einem Behandlungsplan des medizinischen Behandlungssystems. Hierfür kann die Lichtführungseinrichtung beweglich gelagert, insbesondere rotierbar und/oder translatierbar und/oder verschwenkbar, an der Navigationseinheit angeordnet und/oder in die Navigationseinheit integriert sein.

Alternativ oder zusätzlich kann die Lichtführungseinrichtung dazu ausgebildet sein, die vorgegebene Lichtverteilung in Abhängigkeit von mittels des medizinischen Bildgebungsgeräts aufgenommenen medizinischen Bilddaten und/oder von mittels des medizinischen Navigationssystems erfassten Navigationsdaten und/oder von einem Behandlungsplan des medizinischen Behandlungssystems anzupassen. Insbesondere kann die Navigationseinheit dazu ausgebildet sein, insbesondere präoperative und/oder intraoperative, medizinische Bilddaten und/oder einen Planungsdatensatz des Untersuchungsobjekts mit den Navigationsdaten des medizinischen Navigationssystems zu registrieren und den vorgegebenen Pfad an die momentane Positionierung des Untersuchungsobjekts anzupassen. Dabei kann der Planungsdatensatz eine Pfadplanung zur Positionierung des medizinischen Objekts bezüglich des Untersuchungsobjekts aufweisen.

Die Erfindung betrifft in einem dritten Aspekt ein Verfahren zum Ausrichten eines medizinischen Objekts mittels einer vorgeschlagenen Vorrichtung zum Ausrichten eines medizinischen Objekts bezüglich eines Untersuchungsobjekts. Dabei wird die vordefinierte Lichtverteilung mittels der Lichtführungseinrichtung ausgesendet. Ferner wird das Führungselement derart repositioniert, dass die vordefinierte Lichtverteilung die Erkennungsmarke des Hilfselements beleuchtet. Dabei wird das Rotationszentrum auf dem durch die vordefinierte Lichtverteilung vorgegebenen Pfad und von dem Untersuchungsobjekt beabstandet angeordnet. Des Weiteren wird die durch das Führungselement zwangsgeführte Anordnung von Ausrichtelement und Hilfselement um das Rotationszentrum derart repositioniert, dass die vordefinierte Lichtverteilung die Erkennungsmarke mit dem vordefinierten Lichtmuster beleuchtet. Dabei wird die Führungsachse des Ausrichtelements in der vordefinierten Lagebeziehung zu dem Pfad ausgerichtet. Ferner wird das medizinische Objekt entlang der Führungsachse mittels des Ausrichtelements angeordnet und/oder bewegt. Allerdings wird das medizinische Objekt nicht in dem Untersuchungsobjekt bewegt.

Die Vorteile des vorgeschlagenen Verfahrens entsprechen im Wesentlichen den Vorteilen der vorgeschlagenen Vorrichtung zum Ausrichten eines medizinischen Objekts bezüglich eines Untersuchungsobjekts und/oder des vorgeschlagenen Systems. Hierbei erwähnte Merkmale, Vorteile oder alternative Ausführungsformen können ebenso auch auf die anderen beanspruchten Gegenstände übertragen werden und umgekehrt.

In einer weiteren vorteilhaften Ausführungsform des vorgeschlagenen Verfahrens kann das Hilfselement vor dem Repositionieren des Führungselements in der definierten Anordnung bezüglich des Ausrichtelements angeordnet werden. Ferner kann das Hilfselement, nach dem Anordnen des Rotationszentrums auf dem Pfad und vor dem Repositionieren der zwangsgeführten Anordnung, längsgeführt in der definierten Lagebeziehung bezüglich der Führungsachse von der definierten Anordnung in eine weitere Anordnung repositioniert werden.

Ausführungsbeispiele der Erfindung sind in den Zeichnungen dargestellt und werden im Folgenden näher beschrieben. In unterschiedlichen Figuren werden für die gleichen Merkmale die gleichen Bezugszeichen verwendet. Es zeigen:
- Fig. 1: eine schematische Darstellung einer vorteilhaften Ausführungsform einer vorgeschlagenen Vorrichtung zum Ausrichten eines medizinischen Objekts bezüglich eines Untersuchungsobjekts,
- Fig. 2 bis 5: schematische Darstellungen verschiedener Betriebszustände einer weiteren vorteilhaften Ausführungsform einer vorgeschlagenen Vorrichtung zum Ausrichten eines medizinischen Objekts bezüglich eines Untersuchungsobjekts,
- Fig. 6: eine schematische Darstellung einer vorteilhaften Ausführungsform eines vorgeschlagenen Systems,
- Fig. 7: eine schematische Darstellung einer vorteilhaften Ausführungsform eines vorgeschlagenen Verfahrens zum Ausrichten eines medizinischen Objekts.

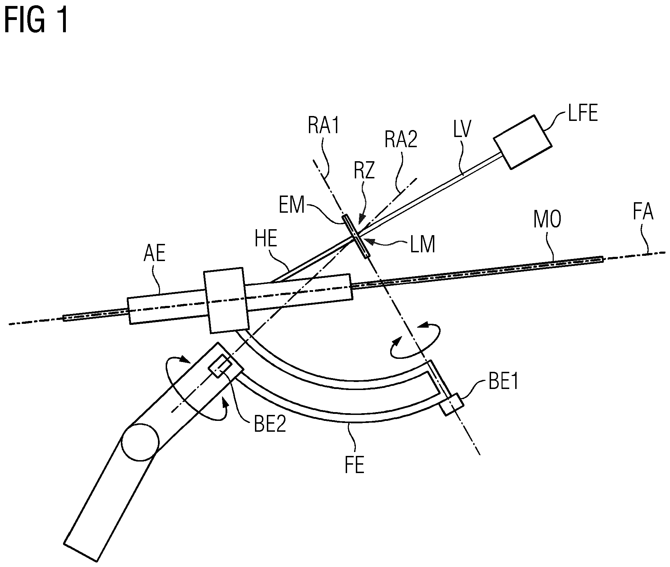

In Fig. 1 ist eine vorteilhafte Ausführungsform einer vorgeschlagenen Vorrichtung zum Ausrichten eines medizinischen Objekts MO bezüglich eines Untersuchungsobjekts schematisch dargestellt. Dabei kann die Vorrichtung ein Ausrichtelement AE, ein Führungselement FE, ein Hilfselement HE und eine Lichtführungseinrichtung LFE umfassen. Das Ausrichtelement AE kann zwangsgeführt durch das Führungselement FE um ein Rotationszentrum RZ herum beweglich gelagert sein. Insbesondere kann das Ausrichtelement AE zwangsgeführt durch das Führungselement FE, um zwei verschiedene Rotationsachsen RA1 und RA2, welche durch das Rotationszentrum RZ verlaufen, beweglich gelagert sein. Das Hilfselement HE kann eine Erkennungsmarke EM aufweisen. Zudem kann das Hilfselement HE in einer definierten Anordnung bezüglich des Ausrichtelements AE derart anordbar sein, dass die Erkennungsmarke EM in dem Rotationszentrum RZ angeordnet ist. Ferner kann die Lichtführungseinrichtung LFE dazu ausgebildet sein, eine vordefinierte Lichtverteilung LV auszusenden, welche einen Pfad vorgibt. Dabei kann die vordefinierte Lichtverteilung LV die Erkennungsmarke EM bei einer von dem Untersuchungsobjekt beabstandeten Anordnung des Rotationszentrums RZ auf dem Pfad beleuchten. Die Anordnung von Ausrichtelement AE und Hilfselement HE kann um das in einem Betriebszustand der Vorrichtung auf dem Pfad angeordnete Rotationszentrum RZ derart bewegbar sein, dass die vordefinierte Lichtverteilung LV die Erkennungsmarke EM mit einem vordefinierten Lichtmuster LM beleuchtet, wenn die Führungsachse FA des Ausrichtelements AE in einer definierten Lagebeziehung bezüglich des Pfads ausgerichtet ist.

Das Ausrichtelement AE kann vorteilhafterweise eine röhrenförmige Hülse aufweisen, welche dazu ausgebildet ist, das medizinische Objekt MO aufzunehmen und entlang der Führungsachse FA zu führen. Ferner kann das Hilfselement HE zumindest einen scheibenförmigen Abschnitt aufweisen. Dabei kann die Erkennungsmarke EM auf einer flachen Seite des zumindest einen scheibenförmigen Abschnitts angeordnet sein und/oder in den zumindest einen scheibenförmigen Abschnitt integriert sein.

Vorteilhafterweise kann die Lichtführungseinrichtung LFE dazu ausgebildet sein, die vordefinierte Lichtverteilung LV aufweisend mehrere Lichtfächer auszusenden, welche nicht parallel zueinander verlaufen und sich entlang des Pfads schneiden.

Die Vorrichtung kann ferner zwei selektiv aktivierbare Feststellelemente BE1 und BE2 umfassen, welche dazu ausgebildet sind, bei Aktivierung das Repositionieren des Rotationszentrums RZ zu sperren und/oder eine Beweglichkeit des Ausrichtelements AE auf eine Rotation um eine der Rotationsachsen RA1 oder RA2, welche durch das Rotationszentrum RZ verlaufen, zu beschränken.

Das Hilfselement HE kann in der definierten Anordnung an dem Ausrichtelement AE befestigt und/oder in das Ausrichtelement AE integriert sein.

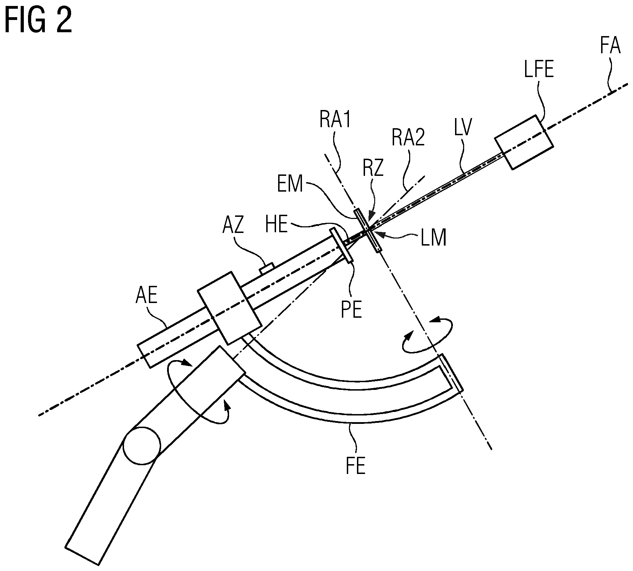

Fig. 2 bis 5 zeigen schematische Darstellungen verschiedener Betriebszustände einer weiteren vorteilhaften Ausführungsform einer vorgeschlagenen Vorrichtung zum Ausrichten eines medizinischen Objekts MO bezüglich eines Untersuchungsobjekts. Dabei kann das Hilfselement HE dazu ausgebildet sein, zumindest teilweise in die Hülse eingeführt zu werden. Ferner kann das Hilfselement HE ein Positionierungsmittel PE aufweisen, welches ein über die definierte Anordnung weitergehendes Einführen des Hilfselements HE in die Hülse blockiert. Vorteilhafterweise kann das Hilfselement HE einen stabförmigen Abschnitt aufweisen, welcher dazu ausgebildet ist, zumindest teilweise in die Hülse eingeführt zu werden. Ferner kann das Hilfselement einen weiteren Abschnitt aufweisen, welcher das Positionierungsmittel PE des Hilfselements HE bildet und das über die definierte Anordnung weitergehende Einführen des Hilfselements HE in die Hülse mechanisch blockiert. Des Weiteren kann das Ausrichtelement AE ein Anzeigemittel AZ aufweisen, welches die definierte Anordnung des Hilfselements HE bezüglich des Ausrichtelements AE anzeigt.

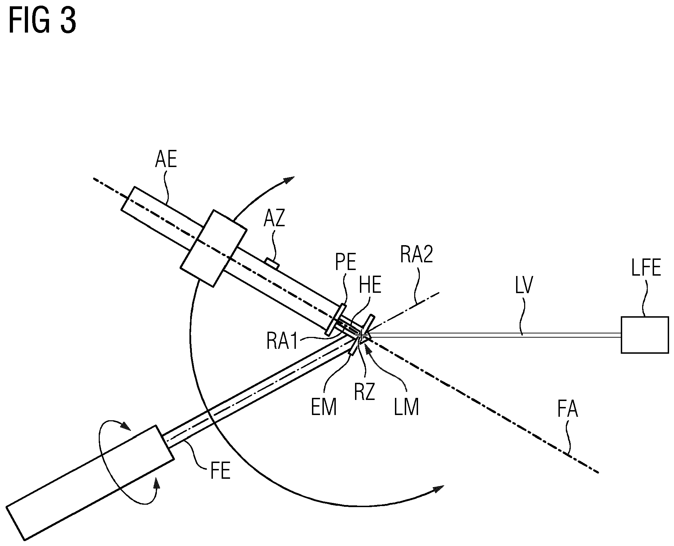

Fig. 2 und 3 zeigen schematische Darstellungen eines Betriebszustands der Vorrichtung aus verschiedenen Sichtwinkeln, in welchem das Hilfselement HE in der definierten Anordnung bezüglich des Ausrichtelements AE angeordnet ist. Dabei kann das Hilfselement HE in der definierten Anordnung zumindest teilweise in die Hülse eingeführt sein. Ferner kann in dem Betriebszustand das Führungselement FE derart positioniert sein, dass das Rotationszentrum RZ auf dem Pfad angeordnet ist. Dabei kann die vordefinierte Lichtverteilung LV die Erkennungsmarke EM beleuchten.

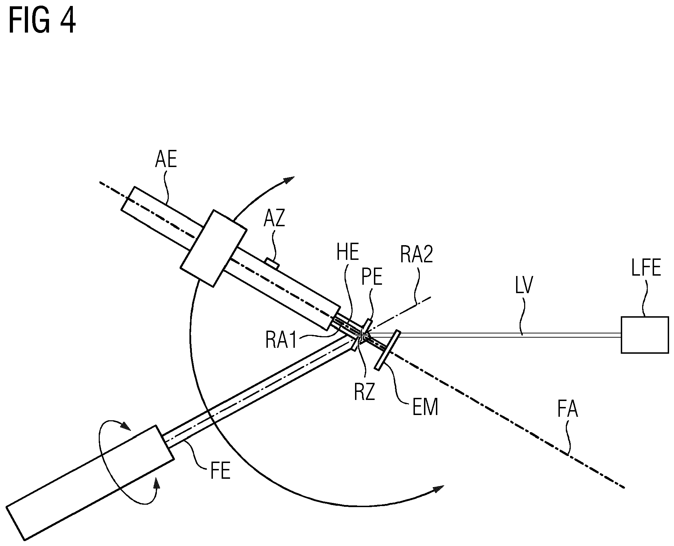

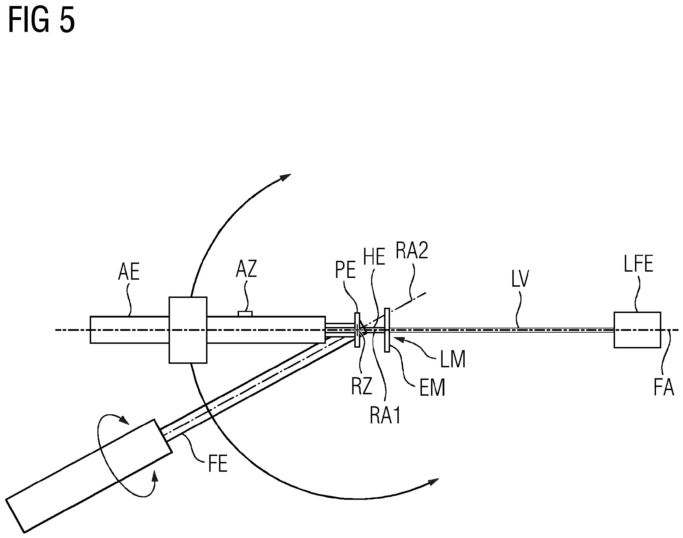

Das Hilfselement HE kann längsgeführt in der definierten Lagebeziehung bezüglich der Führungsachse FA von der definierten Anordnung in eine weitere Anordnung repositionierbar sein. In Fig. 4 ist ein weiterer Betriebszustand der Vorrichtung schematisch dargestellt, in welchem das Hilfselement HE in der weiteren Anordnung bezüglich des Ausrichtelements AE angeordnet ist. Fig. 5 zeigt eine schematische Darstellung eines weiteren Betriebszustands der Vorrichtung, in welchem die weitere Anordnung von Ausrichtelement AE und Hilfselement HE derart um das Rotationszentrum RZ repositioniert wurde, dass die vordefinierte Lichtverteilung LV die Erkennungsmarke EM mit dem vordefinierten Lichtmuster LM beleuchtet. Dabei kann die Führungsachse FA des Ausrichtelements AE in der definierten Lagebeziehung bezüglich des Pfads ausgerichtet sein. Ferner kann dabei die Erkennungsmarke EM an einem weiteren Punkt auf dem durch die Lichtverteilung LV vorgegebenen Pfad angeordnet sein, welcher weitere Punkt verschieden von dem Rotationszentrum RZ ist.

Die Führungsachse FA, das Rotationzentrum RZ, die definierte Anordnung und/oder die weitere Anordnung können vorteilhafterweise in einem Koordinatensystem des Ausrichtelements AE definiert sein, welches sich bei einer Bewegung des Ausrichtelements AE mitbewegt. Zudem kann der durch die Lichtverteilung LV vorgegebene Pfad in einem, insbesondere nicht bewegten, Koordinatensystem des umgebenden Raums, beispielsweise relativ zu dem Untersuchungsobjekt, einer Patientenlagerungsvorrichtung, einer Navigationseinheit und/oder der Lichtführungseinrichtung LFE, definiert sein.

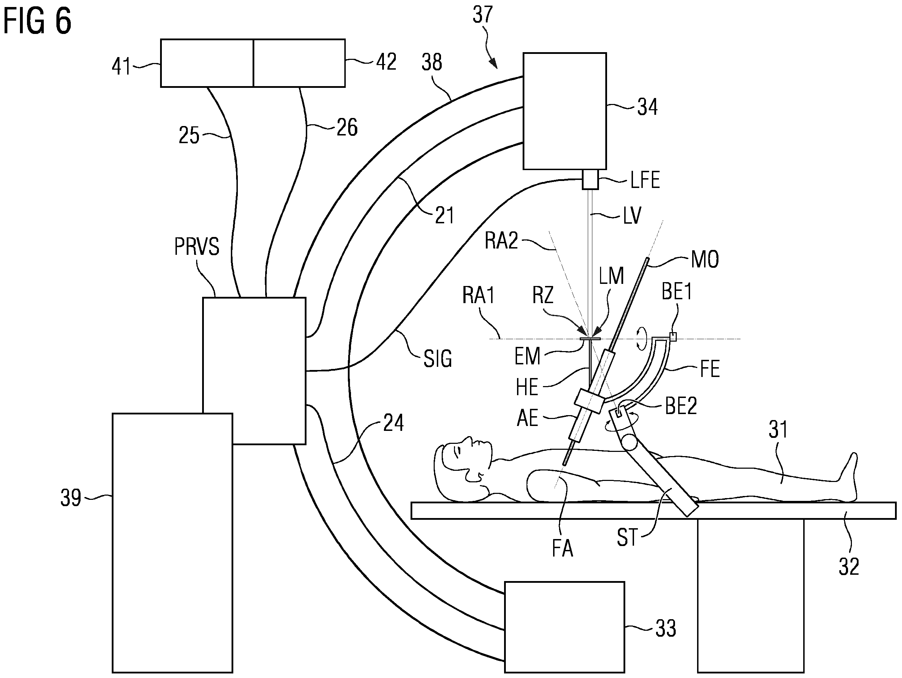

Fig. 6 zeigt eine schematische Darstellung einer vorteilhaften Ausführungsform eines vorgeschlagenen Systems. Dabei kann das System eine vorgeschlagene Vorrichtung zur Ausrichtung eines medizinischen Objekts MO und eine Navigationseinheit umfassen. Dabei kann die Navigationseinheit ein medizinisches Bildgebungssystem und/oder ein medizinisches Navigationssystem und/oder ein medizinisches Behandlungssystem umfassen. In Fig. 6 umfasst die Navigationseinheit beispielhaft für ein medizinisches Bildgebungssystem ein medizinisches C-Bogen-Röntgengerät 37. Die Lichtführungseinrichtung LFE kann vorteilhafterweise an der Navigationseinheit, insbesondere dem medizinischen C-Bogen-Röntgengerät 37, angeordnet und/oder in die Navigationseinheit, insbesondere das medizinische C-Bogen-Röntgengerät 37, integriert sein. Wie in Fig. 6 dargestellt, kann die Lichtführungseinrichtung LFE beispielsweise an einem Detektor 34 des medizinischen C-Bogen-Röntgengeräts 37 angeordnet sein.

Dabei kann das medizinische C-Bogen-Röntgengerät 37 dazu ausgebildet sein, medizinische Bilddaten eines, auf einer Patientenlagerungsvorrichtung 32 angeordneten, Untersuchungsobjekts 31 aufzunehmen und an eine Bereitstellungseinheit PRVS bereitzustellen. Das Führungselement FE kann beispielsweise mittels eines mechanischen Arms und/oder Stativs und/oder Roboterarms ST an der Patientenlagerungsvorrichtung 32 und/oder der Navigationseinheit, insbesondere dem medizinischen C-Bogen-Röntgengerät 37, angeordnet, insbesondere befestigt, sein. Das medizinische C-Bogen-Röntgengerät 37 kann vorteilhafterweise den Detektor 34, insbesondere einen Röntgendetektor, und eine Quelle 33, insbesondere eine Röntgenquelle, aufweisen. Zur Aufnahme der medizinischen Bilddaten kann ein C-Arm 38 des C-Bogen-Röntgengeräts 37 beweglich um ein oder mehrere Achsen herum gelagert sein. Ferner kann das medizinische C-Bogen-Röntgengerät 37 eine Bewegungseinheit 39 umfassen, welche dazu ausgebildet ist, das medizinische C-Bogen-Röntgengerät 37 im Raum zu bewegen. Zur Aufnahme der medizinischen Bilddaten des Untersuchungsobjekts 31, kann die Bereitstellungseinheit PRVS ein Signal 24 an die Röntgenquelle 33 senden. Daraufhin kann die Röntgenquelle 33 ein Röntgenstrahlenbündel aussenden. Beim Auftreffen des Röntgenstrahlenbündels, nach einer Wechselwirkung mit dem Untersuchungsobjekt 31, auf einer Oberfläche des Detektors 34, kann der Detektor 34 ein Signal 21 an die Bereitstellungseinheit PRVS senden. Die Bereitstellungseinheit PRVS kann anhand des Signals 21 die medizinischen Bilddaten empfangen. Die Bereitstellungseinheit PRVS kann ferner dazu ausgebildet sein, die Lichtführungseinrichtung LFE zu steuern, beispielsweise mittels eines Signals SIG. Insbesondere kann die Bereitstellungseinheit PRVS dazu ausgebildet sein, die Lichtführungseinrichtung LFE in Abhängigkeit der medizinischen Bilddaten, insbesondere mittels des Signals SIG, zum Aussenden der vorgegebenen Lichtverteilung LV zu steuern.

Das System kann ferner eine Eingabeeinheit 42, beispielsweise eine Tastatur, und eine Darstellungseinheit 41, beispielsweise einen Monitor und/oder ein Display und/oder einen Projektor, aufweisen. Die Eingabeeinheit 42 kann vorzugsweise in die Darstellungseinheit 41 integriert sein, beispielsweise bei einem kapazitiven und/oder resistiven Eingabedisplay. Die Eingabeeinheit 42 kann vorteilhafterweise zur Erfassung einer Nutzereingabe ausgebildet sein. Hierfür kann die Eingabeeinheit 42 beispielsweise ein Signal 26 an die Bereitstellungseinheit PRVS senden. Die Bereitstellungseinheit PRVS kann dazu ausgebildet sein, das medizinische C-Bogen-Röntgengerät 37 und/oder die Lichtführungseinrichtung LFE in Abhängigkeit der Nutzereingabe, insbesondere in Abhängigkeit des Signals 26, zu steuern. Die Darstellungseinheit 41 kann vorteilhafterweise dazu ausgebildet sein, eine graphische Darstellung der medizinischen Bilddaten anzuzeigen. Hierfür kann die Bereitstellungseinheit PRVS ein Signal 25 an die Darstellungseinheit 41 senden.

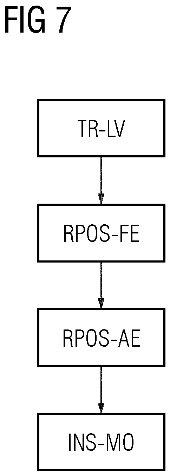

In Fig. 7 ist eine vorteilhafte Ausführungsform eines vorgeschlagenen Verfahrens zum Ausrichten eines medizinischen Objekts MO schematisch dargestellt. Dabei kann die vordefinierte Lichtverteilung LV mittels der Lichtführungseinrichtung LFE ausgesendet werden TR-LV. Ferner kann das Führungselement FE derart repositioniert werden RPOS-FE, dass die vordefinierte Lichtverteilung LV die Erkennungsmarke EM des Hilfselements HE beleuchtet. Dabei kann das Rotationszentrum RZ auf dem durch die vordefinierte Lichtverteilung LV vorgegebenen Pfad und von dem Untersuchungsobjekt (31) beabstandet angeordnet werden. Des Weiteren kann die durch das Führungselement FE zwangsgeführte Anordnung von Ausrichtelement AE und Hilfselement HE derart um das Rotationszentrum RZ repositioniert werden RPOS-AE, dass die vordefinierte Lichtverteilung LV die Erkennungsmarke EM mit dem vordefinierten Lichtmuster LM beleuchtet. Die Führungsachse FA des Ausrichtelements AE kann in der definierten Lagebeziehung zu dem Pfad ausgerichtet werden. Zudem kann das medizinische Objekt MO entlang der Führungsachse FA mittels des Ausrichtelements AE angeordnet und/oder bewegt werden INS-MO.

Vorteilhafterweise kann das Hilfselement HE vor dem Repositionieren RPOS-FE des Führungselements FE in der definierten Anordnung bezüglich des Ausrichtelements AE angeordnet werden. Dabei kann das Hilfselement HE, nach dem Anordnen des Rotationszentrums RZ auf dem Pfad und vor dem Repositionieren RPOS-AE der zwangsgeführten Anordnung, längsgeführt in der definierten Lagebeziehung bezüglich der Führungsachse FA von der definierten Anordnung in die weitere Anordnung repositioniert werden.

Die in den beschriebenen Figuren enthaltenen schematischen Darstellungen bilden keinerlei Maßstab oder Größenverhältnisse ab.

Es wird abschließend noch einmal darauf hingewiesen, dass es sich bei den vorhergehenden detailliert beschriebenen Verfahren und Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "einer" nicht aus, dass die betreffenden Merkmale auch mehrfach vorhanden sein können. Ebenso schließen die Begriffe "Einheit" und "Element" nicht aus, dass die betreffenden Komponenten aus mehreren zusammenwirkenden TeilKomponenten bestehen, die gegebenenfalls auch räumlich verteilt sein können.

## Patentansprüche

1. Vorrichtung zum Ausrichten eines medizinischen Objekts (MO) bezüglich eines Untersuchungsobjekts (31),
umfassend ein Ausrichtelement (AE), ein Führungselement (FE), ein Hilfselement (HE) und eine Lichtführungseinrichtung (LFE),
wobei das Ausrichtelement (AE) zwangsgeführt durch das Führungselement (FE) um ein Rotationszentrum (RZ) herum beweglich gelagert ist,
welches Rotationszentrum (RZ) durch Repositionieren des Führungselements (FE) repositionierbar ist (RPOS-FE),
wobei das Ausrichtelement (AE) zur Längsführung des medizinischen Objekts (MO) entlang einer durch das Ausrichtelement (AE) vorgegebenen Führungsachse (FA) ausgebildet ist,
wobei das Hilfselement (HE) eine Erkennungsmarke (EM) aufweist,
wobei das Hilfselement (HE) in einer definierten Anordnung bezüglich des Ausrichtelements (AE) derart anordbar ist, dass die Erkennungsmarke (EM) in dem Rotationszentrum (RZ) angeordnet ist,
wobei die Lichtführungseinrichtung (LFE) dazu ausgebildet ist, eine vordefinierte Lichtverteilung (LV) auszusenden (TR-LV), welche einen Pfad vorgibt,
wobei die vordefinierte Lichtverteilung (LV) die Erkennungsmarke (EM) bei einer von dem Untersuchungsobjekt (31) beabstandeten Anordnung des Rotationszentrums (RZ) auf dem Pfad beleuchtet,
wobei die Anordnung von Ausrichtelement (AE) und Hilfselement (HE) um das in einem Betriebszustand der Vorrichtung auf dem Pfad angeordnete Rotationszentrum (RZ) derart bewegbar ist, dass die Führungsachse (FA) des Ausrichtelements (AE) in einer definierten Lagebeziehung bezüglich des Pfads ausrichtbar ist, wobei die vordefinierte Lichtverteilung (LV) die Erkennungsmarke (EM) mit einem vordefinierten Lichtmuster (LM) beleuchtet, wenn die Führungsachse (FA) des Ausrichtelements (AE) in der definierten Lagebeziehung bezüglich des Pfads ausgerichtet ist.

2. Vorrichtung nach Anspruch 1,
wobei das Ausrichtelement (AE) eine röhrenförmige Hülse aufweist, welche dazu ausgebildet ist, das medizinische Objekt (MO) aufzunehmen und entlang der Führungsachse (FA) zu führen.

3. Vorrichtung nach Anspruch 1 oder 2,
wobei das Hilfselement (HE) zumindest einen scheibenförmigen Abschnitt aufweist,
wobei die Erkennungsmarke (EM) auf einer flachen Seite des zumindest einen scheibenförmigen Abschnitts angeordnet ist und/oder in den zumindest einen scheibenförmigen Abschnitt integriert ist.

4. Vorrichtung nach einem der vorangehenden Ansprüche,
wobei das Ausrichtelement (AE) zwangsgeführt durch das Führungselement (FE) um zumindest eine Rotationsachse (RA1), welche durch das Rotationszentrum (RZ) verläuft, beweglich gelagert ist.

5. Vorrichtung nach Anspruch 4,
wobei das Ausrichtelement (AE) zwangsgeführt durch das Führungselement (FE) um zwei verschiedene Rotationsachsen (RA1, RA2), welche durch das Rotationszentrum (RZ) verlaufen, beweglich gelagert ist.

6. Vorrichtung nach einem der vorangehenden Ansprüche,
wobei die Lichtführungseinrichtung (LFE) dazu ausgebildet ist, die vordefinierte Lichtverteilung (LV) aufweisend mehrere Lichtfächer auszusenden, welche nicht parallel zueinander verlaufen und sich entlang des Pfads schneiden.

7. Vorrichtung nach einem der vorangehenden Ansprüche, weiterhin umfassend zumindest ein selektiv aktivierbares Feststellelement (BE1, BE2),
welches dazu ausgebildet ist, bei Aktivierung:
- das Repositionieren des Rotationszentrums (RZ) zu sperren
und/oder
- eine Beweglichkeit des Ausrichtelements (AE) auf eine Rotation um eine Rotationsachse (RA1, RA2), welche durch das Rotationszentrum (RZ) verläuft, zu beschränken.

8. Vorrichtung nach einem der vorangehenden Ansprüche,
wobei das Hilfselement (HE) in der definierten Anordnung an dem Ausrichtelement (AE) befestigt und/oder in das Ausrichtelement (AE) integriert ist.

9. Vorrichtung nach einem der Ansprüche 1 bis 7,
wobei das Hilfselement (HE) längsgeführt in der definierten Lagebeziehung bezüglich der Führungsachse (FA) von der definierten Anordnung in eine weitere Anordnung repositionierbar ist,
wobei die weitere Anordnung von Ausrichtelement (AE) und Hilfselement (HE) um das Rotationszentrum (RZ) herum derart bewegbar ist, dass die vordefinierte Lichtverteilung (LV) die Erkennungsmarke (EM) mit dem vordefinierten Lichtmuster (LM) beleuchtet, wenn die Führungsachse (FA) des Ausrichtelements (AE) in der definierten Lagebeziehung bezüglich des Pfads ausgerichtet ist.

10. Vorrichtung nach Anspruch 2 und 9,
wobei das Hilfselement (HE) dazu ausgebildet ist, zumindest teilweise in die Hülse eingeführt zu werden,
wobei das Hilfselement (HE) in der definierten Anordnung zumindest teilweise in die Hülse eingeführt ist.

11. Vorrichtung nach Anspruch 10,
wobei das Hilfselement (HE) und/oder das Ausrichtelement (AE) ein Positionierungsmittel (PE) aufweisen, welches ein über die definierte Anordnung weitergehendes Einführen des Hilfselements (HE) in die Hülse blockiert.

12. Vorrichtung nach Anspruch 11,
wobei das Hilfselement (HE) einen stabförmigen Abschnitt aufweist, welcher dazu ausgebildet ist, zumindest teilweise in die Hülse eingeführt zu werden,
wobei das Hilfselement (HE) einen weiteren Abschnitt aufweist, welcher das Positionierungsmittel (PE) des Hilfselements (HE) bildet und das über die definierte Anordnung weitergehende Einführen des Hilfselements (HE) in die Hülse mechanisch blockiert.

13. Vorrichtung nach einem der vorangehenden Ansprüche,
wobei das Hilfselement (HE) und/oder das Ausrichtelement (AE) ein Anzeigemittel (AZ) aufweisen, welches die definierte Anordnung des Hilfselements (HE) bezüglich des Ausrichtelements (AE) anzeigt.

14. System, umfassend eine Vorrichtung nach einem der vorangehenden Ansprüche und eine Navigationseinheit,
wobei die Navigationseinheit ein medizinisches Bildgebungssystem und/oder ein medizinisches Navigationssystem und/oder ein medizinisches Behandlungssystem umfasst,
wobei die Lichtführungseinrichtung (LFE) an der Navigationseinheit angeordnet und/oder in die Navigationseinheit integriert ist.

15. Verfahren zum Ausrichten eines medizinischen Objekts mittels einer Vorrichtung nach einem der Ansprüche 1 bis 13, umfassend:
- Aussenden (TR-LV) der vordefinierten Lichtverteilung (LV) mittels der Lichtführungseinrichtung (LFE),
- Repositionieren (RPOS-FE) des Führungselements (FE) derart, dass die vordefinierte Lichtverteilung (LV) die Erkennungsmarke (EM) des Hilfselements (HE) beleuchtet,
wobei das Rotationszentrum (RZ) auf dem durch die vordefinierte Lichtverteilung (LV) vorgegebenen Pfad und von dem Untersuchungsobjekt (31) beabstandet angeordnet wird,
- Repositionieren (RPOS-AE) der durch das Führungselement (FE) zwangsgeführten Anordnung von Ausrichtelement (AE) und Hilfselement (HE) um das Rotationszentrum (RZ) derart, dass die vordefinierte Lichtverteilung (LV) die Erkennungsmarke (EM) mit dem vordefinierten Lichtmuster (LM) beleuchtet, wobei die Führungsachse (FA) des Ausrichtelements (AE) in der definierten Lagebeziehung zu dem Pfad ausgerichtet wird,
- Anordnen und/oder Bewegen (INS-MO) des medizinischen Objekts (MO) entlang der Führungsachse (FA) mittels des Ausrichtelements (AE),
wobei das medizinische Objekt (MO) nicht in dem Untersuchungsobjekt (31) bewegt wird.

16. Verfahren nach Anspruch 15 zum Ausrichten des medizinischen Objekts mittels einer Vorrichtung nach einem der Ansprüche 9 bis 12 oder nach Anspruch 9 und 13,
wobei das Hilfselement (HE) vor dem Repositionieren (RPOS-FE) des Führungselements (FE) in der definierten Anordnung bezüglich des Ausrichtelements (AE) angeordnet wird,
wobei das Hilfselement (HE), nach dem Anordnen des Rotationszentrums (RZ) auf dem Pfad und vor dem Repositionieren (RPOS-AE) der zwangsgeführten Anordnung, längsgeführt in der definierten Lagebeziehung bezüglich der Führungsachse (FA) von der definierten Anordnung in eine weitere Anordnung repositioniert wird.

## Claims

1. Device for orienting a medical object (MO) in respect of an object under examination (31),
comprising an orientation element (AE), a guide element (FE), an ancillary element (HE) and a light guiding device (LFE), wherein the orientation element (AE) is movably mounted so as to be forcibly guided by the guide element (FE) about a centre of rotation (RZ),
said centre of rotation (RZ) being repositionable (RPOS-FE) by repositioning the guide element (FE),
wherein the orientation element (AE) is designed for the longitudinal guidance of the medical object (MO) along a guide axis (FA) specified by the orientation element (AE),
wherein the ancillary element (HE) has an identification marker (EM),
wherein the ancillary element (HE) can be arranged in a defined arrangement in respect of the orientation element (AE), such that the identification marker (EM) is arranged in the centre of rotation (RZ),
wherein the light guiding device (LFE) is designed to emit (TR-LV) a predefined distribution of light (LV) which specifies a path,
wherein the predefined distribution of light (LV) illuminates the identification marker (EM) when the centre of rotation (RZ) is arranged on the path at a distance from the object under examination (31),
wherein the arrangement of orientation element (AE) and ancillary element (HE) can be moved about the centre of rotation (RZ) arranged on the path in an operating state of the device, such that the guide axis (FA) of the orientation element (AE) can be aligned in a defined positional relationship in respect of the path, wherein the predefined distribution of light (LV) illuminates the identification marker (EM) with a predefined light pattern (LM), if the guide axis (FA) of the orientation element (AE) is oriented in a defined positional relationship in respect of the path.

2. Device according to claim 1,
wherein the orientation element (AE) has a tubular sleeve which is designed to receive the medical object (MO) and to guide it along the guide axis (FA).

3. Device according to claim 1 or 2,
wherein the ancillary element (HE) has at least one disk-shaped section,
wherein the identification marker (EM) is arranged on a flat side of the at least one disk-shaped section and/or is integrated into the at least one disk-shaped section.

4. Device according to one of the preceding claims,
wherein the orientation element (AE) is movably mounted so as to be forcibly guided by the guide element (FE) about at least one axis of rotation (RA1) which runs through the centre of rotation (RZ).

5. Device according to claim 4,
wherein the orientation element (AE) is movably mounted so as to be forcibly guided by the guide element (FE) about two different axes of rotation (RA1, RA2) which run through the centre of rotation (RZ).

6. Device according to one of the preceding claims,
wherein the light guiding device (LFE) is designed to emit the predefined distribution of light (LV) containing multiple light arrays which do not run parallel to one another and intersect along the path.

7. Device according to one of the preceding claims,
further comprising at least one selectively activatable locking element (BE1, BE2),
which is designed, on activation:
- to block the repositioning of the centre of rotation (RZ)
and/or
- to restrict a movability of the orientation element (AE) to a rotation about an axis of rotation (RA1, RA2) which runs through the centre of rotation (RZ).

8. Device according to one of the preceding claims,
wherein the ancillary element (HE) is attached in the defined arrangement to the orientation element (AE) and/or is integrated into the orientation element (AE).

9. Device according to one of claims 1 to 7,
wherein the ancillary element (HE) can be repositioned in a longitudinally guided manner in the defined positional relationship in respect of the guide axis (FA) from the defined arrangement to a further arrangement,
wherein the further arrangement of orientation element (AE) and ancillary element (HE) can be moved about the centre of rotation (RZ), such that the predefined distribution of light (LV) illuminates the identification marker (EM) with the predefined light pattern (LM), if the guide axis (FA) of the orientation element (AE) is oriented in the defined positional relationship in respect of the path.

10. Device according to claim 2 and 9,
wherein the ancillary element (HE) is designed to be introduced at least partially into the sleeve,
wherein the ancillary element (HE) in the defined arrangement is inserted at least partially into the sleeve.

11. Device according to claim 10,
wherein the ancillary element (HE) and/or the orientation element (AE) have a positioning means (PE) which blocks an introduction of the ancillary element (HE) into the sleeve any further than the defined arrangement.

12. Device according to claim 11,
wherein the ancillary element (HE) has a rod-shaped section which is designed to be introduced at least partially into the sleeve,
wherein the ancillary element (HE) has a further section which forms the positioning means (PE) of the ancillary element (HE) and mechanically blocks the introduction of the ancillary element (HE) into the sleeve any further than the defined arrangement.

13. Device according to one of the preceding claims,
wherein the ancillary element (HE) and/or the orientation element (AE) have a display means (AZ) which displays the defined arrangement of the ancillary element (HE) in respect of the orientation element (AE).

14. System, comprising a device according to one of the preceding claims and a navigation unit,
wherein the navigation unit comprises a medical imaging system and/or a medical navigation system and/or a medical treatment system,
wherein the light guiding device (LFE) is arranged on the navigation unit and/or is integrated into the navigation unit.

15. Method for orienting a medical object by means of a device according to one of claims 1 to 13,
comprising:
- emitting (TR-LV) the predefined distribution of light (LV) by means of the light guiding device (LFE),
- repositioning (RPOS-FE) the guide element (FE), such that the predefined distribution of light (LV) illuminates the identification marker (EM) of the ancillary element (HE), wherein the centre of rotation (RZ) is arranged on the path specified by the predefined distribution of light (LV) and at a distance from the object under examination (31),
- repositioning (RPOS-AE) the arrangement of orientation element (AE) and ancillary element (HE) forcibly guided by the guide element (FE) about the centre of rotation (RZ), such that the predefined distribution of light (LV) illuminates the identification marker (EM) with the predefined light pattern (LM),
wherein the guide axis (FA) of the orientation element (AE) is oriented in the defined positional relationship to the path,
- arranging and/or moving (INS-MO) the medical object (MO) along the guide axis (FA) by means of the orientation element (AE),
wherein the medical object (MO) is not moved in the examination object (31).

16. Method according to claim 15 for orientation of the medical object by means of a device according to one of claims 9 to 12 or according to claim 9 and 13,
wherein the ancillary element (HE) is arranged, prior to the repositioning (RPOS-FE) of the guide element (FE), in the defined arrangement in respect of the orientation element (AE),
wherein the ancillary element (HE), following the arrangement of the centre of rotation (RZ) on the path and prior to the repositioning (RPOS-AE) of the forcibly guided arrangement, is repositioned in a longitudinally guided manner in the defined positional relationship in respect of the guide axis (FA) from the defined arrangement to a further arrangement.

## Revendications

1. Installation d'alignement d'un objet (MO) médical sur un objet (31) à examiner,
comprenant un élément (AE) d'alignement, un élément (FE) de guidage, un élément (HE) auxiliaire et un dispositif (LFE) de guidage de la lumière,
dans laquelle l'élément (AE) d'alignement est monté mobile autour d'un centre (RZ) de rotation à guidage forcé par l'élément (FE) de guidage,
lequel le centre (RZ) de rotation peut être remis en position par la remise en position de l'élément (FE) de guidage (RPOS-FE),
dans laquelle l'élément (AE) d'alignement est constitué pour le guidage longitudinal de l'objet (MO) médical le long d'un axe (FA) de guidage donné à l'avance par l'élément (AE) d'alignement,
dans laquelle l'élément (HE) auxiliaire a un repère (EM) de détection,
dans laquelle l'élément (HE) auxiliaire peut être disposé dans un agencement défini par rapport à l'élément (AE) d'alignement, de manière à ce que le repère (EM) de détection soit disposé au centre (RZ) de rotation,
dans laquelle le dispositif (LFE) de guidage de la lumière est constitué pour envoyer (TR-LV), une répartition (LV) de lumière définie à l'avance, qui prescrit un chemin,
dans laquelle la répartition (LV) de lumière définie à l'avance éclaire sur le chemin le repère (EM) de détection, lorsque le centre (RZ) de rotation est disposé à distance de l'objet (31) à examiner,
dans laquelle l'agencement de l'élément (AE) d'alignement et de l'élément (HE) auxiliaire est mobile autour du centre (RZ) de rotation disposé, lorsque l'installation est dans un état de fonctionnement, sur le chemin de manière à ce que l'axe (FA) de guidage de l'élément (AE) d'alignement puisse être orienté par rapport au chemin en une relation de position définie, dans laquelle la répartition (LV) de lumière définie à l'avance éclaire le repère (EM) de détection par un motif (LM) de lumière défini à l'avance, lorsque l'axe (FA) de guidage de l'élément (AE) d'alignement est orienté par rapport au chemin dans la relation de position définie.

2. Installation suivant la revendication 1,
dans laquelle l'élément (AE) d'alignement a un manchon tubulaire, qui est conformé de manière à recevoir l'objet (MO) médical et à le guider le long de l'axe (FA) de guidage.

3. Installation suivant la revendication 1 ou 2,
dans laquelle l'élément (HE) auxiliaire a au moins une partie en forme de disque,
dans laquelle le repère (EM) de détection est disposé sur une face plane de la au moins une partie en forme de disque et/ou est intégré dans la au moins une partie en forme de disque.

4. Installation suivant l'une des revendications précédentes,
dans laquelle l'élément (AE) d'alignement est monté mobile à guidage forcé par l'élément (FE) de guidage autour d'au moins un axe (RA1) de rotation, qui passe par le centre (RZ) de rotation.

5. Installation suivant la revendication 4,
dans laquelle l'élément (AE) d'alignement est monté mobile à guidage forcé par l'élément (FE) de guidage autour de deux axes (RA1, RA2) de rotation différents, qui passent par le centre (RZ) de rotation.

6. Installation suivant l'une des revendications précédentes,
dans laquelle le dispositif (LFE) de guidage de la lumière est constitué de manière à envoyer la répartition (LV) de lumière définie à l'avance comportant plusieurs éventails de lumière, qui s'étendent les uns par rapport aux autres en n'étant pas parallèles et se coupent le long du chemin.

7. Installation suivant l'une des revendications précédentes, comprenant en outre au moins un élément (BE1, BE2) de fixation activable sélectivement,
qui est constitué pour, lorsqu'il est activé :
- bloquer la remise en position du centre (RZ) de rotation et/ou
- limiter une mobilité de l'élément (AE) d'alignement à une rotation autour d'un axe (RA1, RA2) de rotation, qui passe par le centre (RZ) de rotation.

8. Installation suivant l'une des revendications précédentes,
dans laquelle l'élément (HE) auxiliaire est dans l'agencement défini fixé à l'élément (AE) d'alignement et/ou intégré à l'élément (AE) d'alignement.

9. Installation suivant l'une des revendications 1 à 7,
dans laquelle l'élément (HE) auxiliaire peut, à guidage forcé dans la relation en position définie par rapport à l'axe (FA) de guidage, être remis en position de l'agencement défini dans un autre agencement,
dans laquelle l'autre agencement de l'élément (AE) d'alignement et de l'élément (HE) auxiliaire peut être déplacé autour du centre (RZ) de rotation, de manière à ce que la répartition (LV) de lumière définie à l'avance éclaire le repère (EM) de détection par le motif (LM) de lumière défini à l'avance, lorsque l'axe (FA) de guidage de l'élément (AE) d'alignement est orienté dans la relation de position définie par rapport au chemin.

10. Installation suivant la revendication 2 et 9,
dans laquelle l'élément (HE) auxiliaire est constitué pour être entré au moins en partie dans le manchon,
dans laquelle l'élément (HE) auxiliaire est entré dans l'agencement défini au moins en partie dans le manchon.

11. Installation suivant la revendication 10,
dans laquelle l'élément (HE) auxiliaire et/ou l'élément (AE) d'alignement ont un moyen (PE) de mise en position, qui bloque une entrée de l'élément (HE) auxiliaire dans le manchon allant au-delà de l'agencement défini.

12. Installation suivant la revendication 11,
dans laquelle l'élément (HE) auxiliaire a un tronçon en forme de barreau, qui est constitué pour être entré au moins en partie dans le manchon,
dans laquelle l'élément (HE) auxiliaire a un autre tronçon, qui forme le moyen (PE) de mise en position de l'élément (HE) auxiliaire et qui bloque mécaniquement l'entrée allant au-delà de l'agencement défini de l'élément (HE) auxiliaire dans le manchon.

13. Installation suivant l'une des revendications précédentes,
dans laquelle l'élément (HE) auxiliaire et/ou l'élément (AE) d'alignement ont un moyen (AZ) d'indication, qui indique l'agencement défini de l'élément (HE) auxiliaire par rapport à l'élément (AE) d'alignement.

14. Système, comprenant une installation suivant l'une des revendications précédentes et une unité de navigation,
dans lequel l'unité de navigation comprend un système d'imagerie médicale et/ou un système de navigation médicale et/ou un système de traitement médical,
dans lequel le dispositif (LFE) de guidage de la lumière est monté sur l'unité de navigation et/ou est intégré à l'unité de navigation.

15. Procédé d'alignement d'un objet médical au moyen d'une installation suivant l'une des revendications 1 à 13, comprenant :
- envoyer (TR-LV) la répartition (LV) de lumière définie à l'avance au moyen du dispositif (LFE) de guidage de la lumière,
- remettre (RPOS-FE) en position l'élément (FE) de guidage, de manière à ce que la répartition (LV) de lumière définie à l'avance éclaire le repère (EM) de détection de l'élément (HE) auxiliaire,
dans lequel le centre (RZ) de rotation est disposé sur le chemin donné à l'avance par la répartition (LV) de lumière définie à l'avance et à distance de l'objet (31) à examiner,
- remettre (RPOS-AE) en position l'agencement à guidage forcé par l'élément (FE) de guidage de l'élément (AE) d'alignement et de l'élément (HE) auxiliaire autour du centre (RZ) de rotation, de manière à ce que la répartition (LV) de la lumière définie à l'avance éclaire le repère (EM) de détection par le motif (LM) défini à l'avance,
dans lequel l'axe (FA) de guidage de l'élément (AE) d'alignement est orienté dans la relation en position définie par rapport au chemin,
- mettre et/ou déplacer (INS-MO) l'objet (MO) médical le long de l'axe (FA) de guidage au moyen de l'élément (AE) d'alignement,
dans lequel l'objet (MO) médical n'est pas déplacé dans l'objet (31) à examiner.

16. Procédé suivant la revendication 15 d'alignement de l'objet médical au moyen d'une installation suivant l'une des revendications 9 à 12 ou suivant la revendication 9 et 13,
dans lequel on met l'élément (HE) auxiliaire, avant la remise (RPOS-FE) en position de l'élément (FE) de guidage, dans l'agencement défini par rapport à l'élément (AE) d'alignement,
dans lequel l'élément (HE) auxiliaire, après avoir mis le centre (RZ) de rotation sur le chemin et avant de remettre (RPOS-AE) en position l'agencement à guidage forcé, est remis en position en étant guidé longitudinalement par la relation de position définie par rapport à l'axe (FA) de guidage en un agencement autre que l'agencement défini.
